# EUROPEAN PATENT APPLICATION

(11) **EP 4 060 339 A1**
(43) Date of publication of application: **21.09.2022**
(21) Application number: 21305744.1
(22) Date of filing: 03.06.2021
(51) Int. Cl.: G01N 33/533, B82Y 30/00, G01N 21/27

(54) **METHODS FOR SENSING, MEASURING FREE RADICALS, AND ASSESSING ANTIOXIDANT EFFICACY OF A MOLECULE IN A CELL, AND FLUORESCENT PROBES USED THEREIN**

(30) Priority: 18.03.2021 EP 21305333
(71) Applicant: Wainvam-E, 56270 Ploemeur (FR)
(72) Inventor: BOURNIGAULT-NUQUET, Aurore, 75013 PARIS (FR); RANI, Dipti, 56100 LORIENT (FR); GEIGER, Remi, 56530 QUEVEN (FR)
(74) Representative: Novagraaf Technologies

(57) **Abstract**

The present invention relates to a method for assessing antioxidant efficacy of one or more molecule(s) introduced inside or in the vicinity of a cell by measuring, in said cell, free radical concentration, the method comprising the steps of:
- Functionalizing nanodiamonds having Nitrogen-Vacancy (NV) centers with species able to target an organelle within the cell,
- Introducing the functionalized nanodiamonds inside the cell,
- Illuminating NV centers with green light, and
- Measuring red fluorescence of the NV centers in the cell after the action of the molecule(s), and also optionally before and/or during the action of the molecule(s),
wherein red fluorescence of the NV centers depends on the free radical concentration in the cell.

The present invention also relates to a nanodiamond (ND) having Nitrogen-Vacancy (NV) centers with species able to target an organelle within a cell, said species being chosen as COOH terminating said nanodiamonds linked covalently via amide linkage either with transactivating transcriptional activator (TAT) peptide or with triphenyl phosphonium (TPP) tags.

The present invention further relates to a process for preparing the nanodiamond (ND), and to the use of the nanodiamond (ND) for sensing qualitatively or quantitatively free radicals in a cell sample.

## Description

### Technical field

The present invention refers to a method for assessing antioxidant efficacy of one or more molecule(s) introduced inside or in the vicinity of a cell by measuring, in said cell, free radical concentration.

Therefore, the present invention has utility in biology applications, in particular in cosmetic, pharmaceutic, ecotoxicology and food industries fields.

In the description below, the references into brackets ([ ]) refer to the listing of references situated at the end of the text.

### Background of the Invention

Free radicals are molecules possessing an unpaired electron. Their formation occurs continuously in cells, dominantly in mitochondria, during biochemical reactions involved for example in the respiratory chain and in phagocytosis. However, many free radicals are unstable and highly reactive species and can be harmful in some cases. In particular, when they are present in excess, they may attack some relevant molecules in the cell such as DNA, proteins and lipids, leading to cell damage and homeostatic disruption. Antioxidants are naturally produced by the cell to avoid this excess and maintain an equilibrium. They are able to neutralize free radicals via accepting or donating electron(s) to remove the unpaired status of the radical so it becomes less active, less dangerous, and long-lived stable substance.

Oxidative stress corresponds to an imbalance between free radicals quantity and antioxidant defenses production. It implies several kinds of damage in cells on a wide range of biochemical species such as lipids, proteins and nucleic acids. Oxidative stress can be short-termed in case of heat injury, intoxication or excessive exercise for example. A long term oxidative stress leads to serious changes in the structure and function of lipids and proteins. This is why oxidative stress is implied in numerous diseases including cancers, inflammatory diseases (as arthritis, vasculitis, etc.), ischemic diseases (as heart diseases, stroke, etc.), neurological disorders (as Alzheimer's disease, Parkinson's disease, etc.), diabetes, and many others.

Skin, the largest tissue in the human body, serves as the interface with the external world, like a protection layer. In addition to endogenous production of free radicals, it undergoes externally generated sources, such as cigarette smoke, air and water pollution, solar radiation. It though particularly suffers from oxidative stress. This oxidative stress encourages some benign dysfunctions, like dark spots, loss of elasticity, apparition of wrinkles, or more serious diseases such as fibrosis and skin cancer.

Numerous cosmetic products use antioxidant molecules to cope with oxidative stress in skin. Among them, famous ones are vitamins, polyphenols and carotenoids. New ones are discovered regularly, often from natural extracts. Testing the efficacy of these molecules is important to offer reliable products. An efficient antioxidant scavenges free radicals, so decreases the free radical concentration. As a consequence, the balance is recovered and the cell is not damaged anymore.

Several techniques exist to assess oxidative stress in vitro. Most methods are indirect, which means that they measure the effect of oxidative stress on living cells or extracted molecules. These methods mainly use organic fluorophores to mark cell reaction to oxidative stress, like the overproduction of some enzymes, protein damage, lipid peroxidation, etc. The marked cells are then analyzed with flow cytometry, colorimetry, chromatography or fluorescence microscopy. There are some limits to these indirect methods. First, they give information about the history, what have happened in the cell, but not the current situation of the free radical content. Moreover, the markers are consumed during the analysis. Finally, the fluorescence of these fluorophores depends a lot on the environment (temperature and pH) and the molecules photobleach very quickly. Because of that, kinetics over several hours or days cannot be performed on the same sample and the results can only be semi-quantitative (this means that they can be compared relatively to each other, and a calibration curve is required to get a quantitative measurement). These indirect methods may be used to evaluate antioxidant efficacy, characterizing oxidative stress before and after antioxidant treatment. However, the results are not completely quantitative and reproducible, mainly because of the marker photobleaching and because every cell is different.

Some similar markers have been developed to detect directly the free radicals, in cells or outside cells. The marker is incorporated in the sample and its colour or fluorescence changes whether it is oxidized by free radicals or not. The colour change is recorded thanks to spectrophotometry, microscopy or cytometry. Some particular protocols have even been adapted to efficacy tests of antioxidants. ORAC (oxygen radical absorbance capacity), ABTS (2,2'-azinobis-(3-ethylbenozothiazoline-6- sulfonate)) and DPPH (1,1-diphenyl-2-picrylhydrazyl) can be cited as examples. In these protocols, free radicals are generated by a chemical reaction or a chain of reactions or physical phenomena (with temperature or light). Then, the antioxidant is added in the medium and the colour or fluorescence of the probe is recorded over time. Once again, these probes photobleach and their signal often depends on the environment so the measurement is only semi-quantitative. Furthermore, the reaction with the free radicals is not completely specific, other parameters or molecules may bias the signal.

Some spectrometry measurement enable to detect and quantify free radicals, such as EPR (Electron Paramagnetic Resonance) and NMR (Nuclear Magnetic Resonance). EPR is specifically designed to quantitatively measure unpaired electrons but it is complicated to use and expensive. First, this technology requires large and powerful magnets (in the range of Teslas) to reach a sufficient sensitivity. In addition, it cannot be performed on living cells or tissues because of numerous restraints such as the cooling of biological samples with liquid nitrogen to slow the cross-reaction of free radicals.

Thus, a need exists of alternative methods, especially methods that would be quantitative and reproducible on different kinds of cells, and that would have a sufficient sensitivity. The present invention fulfills these and other needs.

### Description of the invention

After extensive researches, the Applicant has managed to develop a solution for antioxidant efficacy testing and/or oxidative stress level measurement, that enables to quantify free radical concentration, notably including fast ones, with possible quantification over time in cells.

The Applicant proposes a method for assessing antioxidant efficacy for products including detection and quantification of free radical concentration in cells, possibly before during and after the action of such products.

This technology surprisingly makes it possible to target cell organelles where the free radicals are mainly generated, especially mitochondria, to be located as close as possible to the generation of majority of free radicals and by this means to reach high sensitivity.

The Applicant managed to develop a probe allowing to reach these goals, which is a fluorescent nanodiamond functionalized to reach specific organelles, as mitochondria. The invention is based on the quantification of fluorescence of Nitrogen-Vacancy (NV) centers in nanodiamonds functionalized with species able to target organelles in cells, especially skin cells.

Accordingly, in a first aspect, the present invention provides a method for assessing antioxidant efficacy of one or more molecule(s) introduced inside or in the vicinity of a cell by measuring, in said cell, free radical concentration, the method comprising the steps of:
- Functionalizing nanodiamonds having Nitrogen-Vacancy (NV) centers with species able to target an organelle within the cell,
- Introducing the functionalized nanodiamonds inside the cell,
- Illuminating NV centers with green light, and
- Measuring red fluorescence of the NV centers in the cell after the action of the molecule(s), and also optionally before and/or during the action of the molecule(s),
wherein red fluorescence of the NV centers depends on the free radical concentration in the cell.

"Nanodiamonds" refers herein to diamonds having nanoscale. Advantageously, the nanodiamonds may have a size included between 5nm and 150nm. Advantageously, the nanoscale of the nanodiamonds allows them to be internalized and/or located inside cells, in particular living cells, without disturbing the cell homeostasis. Another advantage of nanodiamonds is their biocompatibility, which mean the viability of the cell and the production of reactive oxygen species are not modified when the nanodiamond is uptaken by the cell.

"Nitrogen-Vacancy (NV) centers" refers herein to defects that are artificially created in nanodiamonds. A carbon atom in the diamond's crystal lattice is replaced by a nitrogen atom (defect, N), and a neighbouring lattice site is left empty (vacancy, V) to form NV center. NV centers may be detected by any methods of the state of the art known for this purpose. These defects exhibit red fluorescence properties under green light excitation. The fluorescence depends on environment, for example the external magnetic field. Free radicals, responsible for oxidative stress, generate a magnetic field in a random direction thanks to the unpaired electron. The production and reaction of free radicals happen very quickly so that the concentration generally remains in stationary state. This magnetic noise is linked to free radical concentration. NV centers have also very high photostability of their red fluorescence. Thanks to the biocompatibility and the photostability of nanodiamonds, it is possible to make long term measurements with more reliability on any given sample. This is how the concentration of free radicals can be measured in real time in a given sample, advantageously before, during and after the action of one or more antioxidant molecule(s). As further explained below, the results may be processed and quantitatively linked to free radical concentration and antioxidant efficacy.

Linkage of some species at the surface of fluorescent nanodiamonds (FNDs), called as surface functionalization, is useful to localize them at or in close vicinity to target location inside cells. This functionalization may have several advantages. It may limit the aggregation of the FNDs, help to enter specific cells, such as skin cells, and/or enable targeting specifically and univocally the elements of interest. Thus, the nanodiamonds can be specifically located at or in close vicinity to specific organelles, especially mitochondria as it is the organelle where the free radicals are mainly generated, to detect free radicals in cells. In an additional aspect, the nanodiamonds may be customized to enhance their uptake by specific cells, for example skin cells. Advantageously, functionalization of the surface of nanodiamonds may be realized by any method known in the art for this purpose. The functional groups may be for example OH or COOH, and be present at the surface of fluorescent nanodiamonds due to chemical treatment. Advantageously, they allow covalent binding of organelles targeting molecules, as peptides or lipophilic cations, which is controlled, in comparison to physiosorbed molecules. For example, functionalization may be realized on the surface carboxyl groups of the nanodiamonds, activated using N-ethyl-N'-(-3-dimethylaminopropyl) carbodiimide hydrochloride (EDC) and N-hydroxysuccinimide (NHS) or Sulfo-NHS.

The species able to target an organelle within the cell may be any species, as chemicals, or biomolecules, known in the art to target at least one of the organelles of the cells. Advantageously, the organelle may be mitochondria, to be located as close as possible to the generation of majority of free radicals and by this means to reach great sensitivity. For example, the species may be trans-activating transcriptional activator (TAT) peptide (with amino acid sequence YGRKKRRQRRR, corresponding to SEQ ID NO: 1) or with triphenyl phosphonium (TPP) tags. In this embodiment, the activated surface of nanodiamonds, as the activated COOH terminating groups, may be reacted with TAT peptide or with TPP tags, for example by amide covalent linkage. An example of process for preparing nanodiamonds of the invention will be described below.

In the method of the invention, introducing the functionalized nanodiamonds inside the cell may be realized by any method known in the state of the art for this purpose. This can be performed *in vitro,* for example by incubating the functionalized nanodiamonds in cell medium with the cell for a period and under conditions allowing their uptake inside the cell, or by physical or chemical permeabilization of the cell membrane. Period and conditions of culture may be determined by the skilled person in view of his technical skills, notably depending on the kind of cell.

Illuminating NV centers with green light may be realized at a wavelength of between 500 and 550 nm, for example about 532 nm. The light source may be directly pulsed or continuous and completing with a modulating device. Advantageously, when they are illuminated with green light, FNDs emit a red fluorescence that may be measured with any mean known in the state of the art, for example an optical detector. These fluorescence properties (optical spectra of FND excitation and emission) are known in the state of the art. The optical intensity of red luminescence depends on the quantum electron spin state in which the NV center is located: the null electron spin state is more luminescent than non-zero electron spin states. The distribution of spin states in the fundamental state, and thus the level of fluorescence, depends on the magnetic environment. When magnetic field varies randomly temporally and spatially, it is called magnetic noise. To detect it, any mean known in the state of the art for this purpose may be used; for example a relaxometry protocol may be performed.

To reliably assess the antioxidant efficacy, the oxidative stress is commonly characterized before, during and after the action of the antioxidant molecule(s). Several parameters are to be considered:
1. measuring the free radical concentration. This is a technical challenge because they are very reactive species, which means they are very short lived (from ns to ms) and present in very low concentration in cells (from nanomolar (nM) to micromolar (µM))
2. obtaining an absolute and quantitative measurement
3. being able to measure kinetics on a given sample to improve reproducibility
4. using a biocompatible system, not to disturb the cell homeostasis
5. sensing close by the mitochondria, dominating organelle for generation of free radicals, to be sensitive enough.

Advantageously, the measurement of red fluorescence of the NV centers in the cell may be carried out before and after the action of the molecule(s), or may be carried out during and after the action of the molecule(s), or may be carried out before, during and after the action of the molecule(s).

Advantageously, the step of measuring red fluorescence of the NV centers in the cell may be repeated over time to follow a kinetic reaction.

Advantageously, the red fluorescence signal detected may be measured at the very beginning of read out pulse, for example in the first 200ns to 500ns, to read the spin state. This signal may be analysed by any method known in the art. For example, the signal may be normalized to allow the comparison between different measurements. Advantageously, to refine the link between free radical concentration and red fluorescence signal, further characterization can be considered. Advantageously, other options are to be considered for data processing. For instance, thanks to imaging part and particle tracking algorithm, it is possible to spatially follow the fluorescent probe inside the cell.

From the quantitative measurement of fluorescence, it is possible to propose criteria to characterize reliably antioxidant efficacy. One example is the ratio R = C_{f}/C₀*100 where C_{f} is the final concentration and Co the initial concentration of free radicals in the cell. This parameter allows to classify the molecules, according to thresholds that can be chosen by the user depending on the type of cell, the molecule(s) and/or the type of organelle. Another example uses the kinetics. Considering the free radical concentration evolution over time, it is possible to measure the antioxidant reaction speed and assess the scavenging efficacy of the antioxidant. A classifying parameter could be the time needed to reach the final concentration. Advantageously, thanks to a temporal resolution and a remarkable photostability, kinetics can be performed to evaluate antioxidant activity along time, from seconds to hours and days.

According to the invention, the at least one molecule that are challenged for their oxidant or antioxidant properties may be any molecule likely to be contacted with cells. The at least one molecule may be for example 1 molecule, or a 2 molecules, or 3 molecules, or more. When more than one molecule is used, they may be of the same type, i.e. they may belong to the same functional or structural family. Alternatively, all or part of the molecules may be of different types, i.e. they may belong to a different functional or structural family. The molecule(s) may be for example at least one vitamin (e.g. E, C, A), and/or at least one carotenoid, and/or at least one polyphenol, as flavonoids. These molecules can be used in different fields, for different applications.

The cell may be a living cell or a lysed cell. The cell may be of any kind. For example, it may be a cell of skin, as fibroblasts or keratinocytes, liver, kidney, lung, uterus, prostate, testis, tongue, bone marrow, colon, bronchi, muscle, pancreas, brain, eye, blood, urinary bladder or of gallbladder. Advantageously, the probe, methods and protocols described above can be adapted to any cells.

According to the invention, cell sample may be cultured and proliferate *in vitro* before introducing the functionalized nanodiamonds inside the cell. Any cellular culture known by the skilled person for this purpose may be carried out, as for example the one described by Vangipuram et al. ("Skin punch biopsy explant culture for derivation of primary human fibroblasts". J Vis Exp. 2013 Jul 7;(77):e3779. doi: 10.3791/3779. PMID: 23852182; PMCID: PMC3731437 ([1])). Then, the cells may be cultured with the at least one molecule for a period of time conducive to the evolution of free radicals in the cells. This period of time may be determined by the skilled person in view of the kind of cell and molecule.

Another object of the invention relates to a device comprising a processor and a memory, said memory comprising code that, when executed by said processor, causes the device to perform the method as described above.

When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), Graphical Processing Unit (GPU), read only memory (ROM) for storing software, random access memory (RAM), and non-volatile storage. Other hardware, conventional or custom, may also be included. Their function may be carried out through the operation of program logic, through dedicated logic, through the interaction of program control and dedicated logic, or even manually, the particular technique being selectable by the implementer as more specifically understood from the context.

According to the invention, memory may include or store computer program code, and the memory and the computer program code may be configured to, with the processor, cause a network element or network device to perform the necessary tasks. Additionally, the processor, memory and example algorithms, encoded as computer program code, serve as means for providing or causing performance of operations discussed herein.

In another aspect, the invention relates to a nanodiamond (ND) having Nitrogen-Vacancy (NV) centers with species able to target an organelle within a cell, said species being chosen as COOH terminating said nanodiamonds linked covalently via amide linkage either with trans-activating transcriptional activator (TAT) peptide or with triphenyl phosphonium (TPP) tags.

Another object of the invention relates to a process for preparing a nanodiamond (ND) as described above, wherein the species is COOH terminating said nanodiamonds linked covalently with trans-activating transcriptional activator (TAT) peptide via amide linkage, said process comprising the steps of:
A1) activating the surface carboxyl groups of the nanodiamond using N-ethyl-N'-(-3-dimethylaminopropyl) carbodiimide hydrochloride (EDC) and N-hydroxysuccinimide (NHS) or Sulfo-NHS, and
A2) reacting the activated carboxyl groups with NH₂ terminated TAT peptide to form TAT-ND via amide linkage between the carboxyl and amine groups,
OR
B1) activating the surface carboxyl groups of the nanodiamond using N-ethyl-N'-(-3-dimethylaminopropyl) carbodiimide hydrochloride (EDC) and N-hydroxysuccinimide (NHS) or Sulfo-NHS,
B2) reacting activated carboxyl groups with PEG-diamine to form NH2-PEG-ND, and
B3) activating carboxyl groups in TAT peptide using EDC, NHS or sulfo-NHS and reacting them with NH2-PEG-ND to form TAT-PEG-ND.

Another object of the invention relates to a process for preparing a nanodiamond (ND) as described above, wherein the species is COOH terminating said nanodiamonds linked covalently with triphenyl phosphonium (TPP) tags, said process comprising the steps of:
C1) activating the surface carboxyl groups of ND using N-ethyl-N'-(-3-dimethylaminopropyl) carbodiimide hydrochloride (EDC) and N-hydroxysuccinimide (NHS) or Sulfo-NHS,
C2) reacting said activated carboxyl groups with NH₂ terminated TPP tag to form ND-TPP via amide linkage.

In another aspect, the invention relates to the use of a nanodiamond (ND) as defined above for sensing qualitatively or quantitatively free radicals in a cell sample which can be living or lysed.

This invention is further illustrated by the following examples with regard to the annexed drawings that should not be construed as limiting.

### Brief description of the figures

- Figure 1: represents a schematic illustration of covalent linkages of mitochondria targeting molecules (TAT peptide and TPP tag) at carboxylated FNDs. A: Amine terminated TAT peptide linkage. B: TAT peptide linkage. C: TPP tag linkage.
- Figure 2: represents raw results of relaxometry measurement: fluorescence decay curve. The inset represents pulse sequence for relaxometry protocol.
- Figure 3: represents sketch of the set up to measure relaxation time T1 of NV centers in nanodiamonds.
- Figure 4: represents Fluorescence decay curve for bare ND outside cells (__), functionalized NDs outside cells (X) and functionalized ND inside cells ( 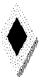 ).
- Figure 5: represents the fluorescence decay curve before ( 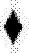 ), during and after antioxidant action.
- Figure 6: represents the kinetic evolution of free radical concentration.

### Examples

### Example 1: fluorescent nanodiamonds (FND) functionalization with TAT peptide: Amine terminated TAT peptide linkage

To carry out the surface functionalization of FNDs, the following protocol is carried out.

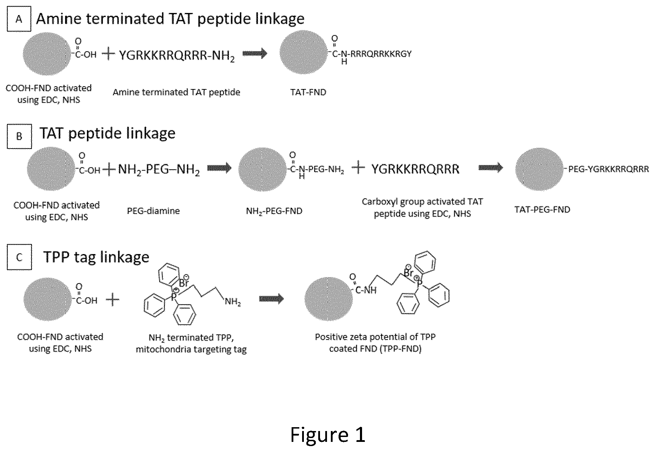

As illustrated in figure 1A, the surface carboxyl groups of FNDs are activated using N-ethyl-N'-(-3-dimethylaminopropyl) carbodiimide hydrochloride (EDC) and N-hydroxysuccinimide (NHS) or Sulfo-NHS. The activated carboxyl groups are made to react with NH₂ terminated TAT peptide to form TAT-FND via amide linkage between the carboxyl and amine groups.

The size and Zeta potential of the FNDs are characterized before and after TAT molecules functionalization using systems such as Zetasizer. Also, the size of FNDs will be confirmed with scanning electron microscopy. Owing to the presence of a number of arginines in TAT peptide which are positively charged at neutral / acidic conditions, TAT functionalized FNDs have positive Zeta potential. The positive Zeta potential of FNDs conjugated with TAT peptide molecules enhances their uptake into mitochondria owing to its highly negative inner membrane potential.

Moreover, other techniques such as photoluminescence spectrophotometer are used to study the emission properties of FNDs to confirm negligible influence of functionalization on emission of NVs. The chemical bonds formed on functionalization of FNDs are confirmed using Fourier transform infrared spectroscopy (FTIR) and elemental composition is evaluated using X-ray photoelectron spectroscopy (XPS).

In summary, the peptide coating results in positive zeta potential of the functionalized FNDs, thus provides electrostatic attracting force towards negative potential of the cellular and mitochondrial membranes. The TAT peptide functionalization of FNDs enhances their cellular uptake and targeting of mitochondria. Moreover, the TAT peptide does not increase the size of TAT functionalized FNDs to a large extent. This is beneficial for their uptake inside mitochondria.

### Example 2: fluorescent nanodiamonds (FND) functionalization with TAT peptide: TAT peptide linkage

To carry out the surface functionalization of FNDs, the following protocol is carried out.

As illustrated in figure 1B, the surface carboxyl groups of FNDs are activated using N-ethyl-N'-(-3-dimethylaminopropyl) carbodiimide hydrochloride (EDC) and N-hydroxysuccinimide (NHS) or Sulfo-NHS. Then, the activated surface carboxyl groups of FNDs are reacted with polyethylene glycol diamine (NH2-PEG-NH2, used as a linker) to form NH₂-PEG-FND. Thereafter, the carboxyl groups in TAT peptide are activated using EDC, NHS (or sulfo-NHS) and are reacted with NH₂-PEG-FND to form TAT-PEG-FND.

The size and Zeta potential of the FNDs are characterized before and after TAT molecules functionalization using systems such as Zetasizer. Also, the size of FNDs will be confirmed with scanning electron microscopy. Owing to the presence of a number of arginines in TAT peptide which are positively charged at neutral / acidic conditions, TAT functionalized FNDs have positive Zeta potential. The positive Zeta potential of FNDs conjugated with TAT peptide molecules enhances their uptake into mitochondria owing to its highly negative inner membrane potential.

Moreover, other techniques such as photoluminescence spectrophotometry are used to study the emission properties of FNDs to confirm negligible influence of functionalization on emission of NVs. The chemical bonds formed on functionalization of FNDs are confirmed using Fourier transform infrared spectroscopy (FTIR) and elemental composition is evaluated using X-ray photoelectron spectroscopy (XPS).

### Example 3: fluorescent nanodiamonds (FND) functionalization with TPP tag

To carry out the surface functionalization of FNDs, the following protocol is carried out.

As illustrated in figure 1C, the surface carboxyl groups of FNDs are activated using N-ethyl-N'-(-3-dimethylaminopropyl) carbodiimide hydrochloride (EDC) and N-hydroxysuccinimide (NHS) or Sulfo-NHS and allowed to react with NH2 terminated TPP tag to form FND-TPP via amide linkage.

The size and Zeta potential of the FNDs are characterized before and after TPP molecules functionalization using systems such as Zetasizer. Also, the size of FNDs will be confirmed with scanning electron microscopy. Conjugation of FNDs with liphophilic cations such as TPP, results in positive Zeta potential of FNDs. The positive Zeta potential of FNDs conjugated with TPP molecules enhances their uptake into mitochondria owing to its highly negative inner membrane potential.

Moreover, other techniques such as photoluminescence spectrophotometer are used to study the emission properties of FNDs to confirm negligible influence of functionalization on emission of NVs. The chemical bonds formed on functionalization of FNDs are confirmed using Fourier transform infrared spectroscopy (FTIR) and elemental composition is evaluated using X-ray photoelectron spectroscopy (XPS).

In summary, conjugation of FNDs with lipophilic cations such as TPP enhances their uptake into mitochondria.

### Example 4: assessing antioxidant efficacy of a molecule

After the first step of preparing functionalized FNDs as illustrated in Examples 1, 2 or 3, the following steps are carried out.

### Step 2: Sample preparation

Skin cells, such as fibroblast, are cultured and proliferate. Cellular culture is carried out as described by Vangipuram et al. ([1]). Fluorescent nanodiamonds are uptaken by the skin cells, by incubation in cell medium or by physical or chemical permeabilization of the cell membrane. Viability tests and metabolism assays are performed to check that the cellular homeostasis is not disturbed.

The sample is presented on any biocompatible holder, comprising Petri dishes, microplates or fluidic device.

### Step 3: Measurement

To detect magnetic noise, a relaxometry protocol is performed.

The longitudinal relaxation time T1 corresponds to the time it takes for a system in which some electrons have been polarized in a particular state to return to its equilibrium state. T1 measurement consists of several cycles of three distinct steps :
1. The NV center is excited with green light to polarize the spins of the center NV from equilibrium distribution to the null state of the ground state.
2. The light is then turned off during an extinction time τ. The spins evolve freely and tend to return to their state of equilibrium.
3. A new green light pulse is sent during which the state of the NV center's spins is read out thanks to the red fluorescence signal. The higher the signal, the more the spins are in the null state, which means that they have not had time to return to the state of equilibrium.

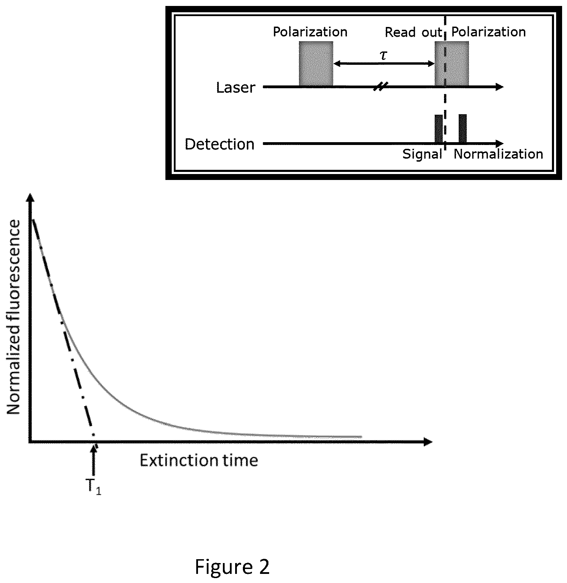

This process is repeated by gradually increasing the extinction time. The obtained curve of fluorescence versus extinction time is used to determine the relaxation time T1, as illustrated on Figure 2. To reduce noise and uncertainty, a decay curve is repeated several times and averaged.

When T1 is long, most of the spins have not been disturbed by an external field and have remained in the null state. When T1 is short, it means that the non-zero spin levels have been populated by an external field, i.e. there are many free radicals. To conclude, the higher the magnetic noise, the shorter the T1. In the present case, the free radicals concentration is linked to magnetic noise measured by T1 relaxometry. The concentration is measured before, during and after the action of an antioxidant.

This protocol allows the measurement of relaxation time between 1µs and 1ms, to detect free radical concentration from nanomolar (nM) to micromolar (µM). It is then a very sensitive technology when others approximatively reach the micromolar level. One measurement can take from few seconds to few minutes according to the aimed sensitivity. The protocol can be repeated over time to follow a kinetic reaction, from seconds to hours and days, on a given sample as long as the cell lives, because the fluorescent nanodiamond does not photobleach, when other technologies can only make before/after measurement.

### Description of the set-up:

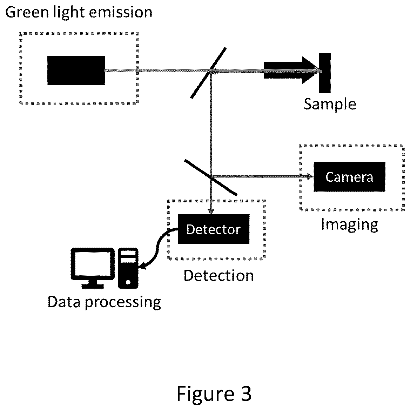

The set-up used to perform this T1 measurement is illustrated on Figure 3. Green light is emitted and pulsed. The wavelength is included between 500 and 550nm. The light source can be directly pulsed or continuous. If continuous, the beam has to be pulsed thanks to another equipment, such as acousto-optic modulator. The pulsing sequence is monitored thanks to programmation and acquisition card.

A lens or an ensemble of lenses such as microscope objective focuses the beam to the sample. The power does not exceed 50µW on the sample to avoid cell damage or stress. This condition is checked simply with a powermeter.

The lens or ensemble of lenses collects the backscattered red fluorescence of the nanodiamonds and collimate the beam towards the detector. A dichroic mirror is used to separate green incident light and red fluorescence. The cut off wavelength of the dichroic mirror is between 550 and 600nm.

The detector is sensitive enough to detect signal from 10pW to 100nW and fast enough to detect pulses separated by 100ns to 10ms. The data are collected thanks to an oscilloscope to extract the fluorescence decay curve. A fitting algorithm extracts the relaxation time T1.

It is possible to add some elements for imaging the sample. For example, a wide field imaging system can be easily installed. A confocal set-up can also be considered, using a scanning device, such as a galvo mirror.

Each element can be set up in free space or connected with optical fibers.

### Step 4: Data processing

The fluorescence signal detected on the photodetector is measured at the very beginning of step 3 of the cycle, in the first 200ns to 500ns, to read the spin state. This signal is normalized to allow the comparison between different measurements.

The experimentally obtained curve of fluorescence decay (Figure 2) is fitted with an exponential model. From this fit, a characteristic time, that is relaxation time T1, is extracted.

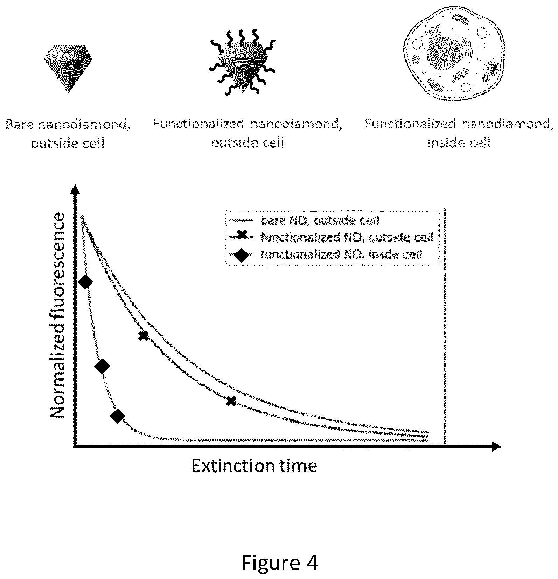

The measured T1 is linked to free radical concentration. This link is determined with a physical model (Tetienne, J. P., Hingant, T., Rondin, L., Cavailles, A., Mayer, L., Dantelle, G., & Jacques, V. (2013). Spin relaxometry of single nitrogen-vacancy defects in diamond nanocrystals for magnetic noise sensing. Physical Review B, 87(23), 235436 ([2])) or/and thanks to an experimental calibration curve (Martinez, F. P., Nusantara, A. C., Chipaux, M., Padamati, S. K., & Schirhagl, R. (2020). Nanodiamond Relaxometry-Based Detection of Free-Radical Species When Produced in Chemical Reactions in Biologically Relevant Conditions. ACS sensors, 5(12), 3862-3869 ([3])). To refine this link, further characterization can be considered. For instance, the effect of functionalization on the nanodiamond properties is determined: T1 is measured outside cells before and after the functionalization, as illustrated on Figure 4. This allows to distinguish the impact of functionalization and the impact of free radicals on the measured T1.

### Step 5: Assessment of antioxidant efficacy

From the quantitative measurement described above, we use the ratio R = Cf/C0*100 to characterize reliably antioxidant efficacy, where Cf is the final concentration and C0 the initial concentration of free radicals in the cell. This parameter allows to classify the molecules, according the following table for instance (it being understood that the thresholds/ranges depend on the application envisaged and can be determined by the skilled person in view of his technical knowledge):

| | |
|---|---|
| R > 100% | The molecule generates free radicals. It is possibly toxic and certainly not antioxidant. |
| 100%>R> 50% | The molecule is not a good antioxidant |
| 50%>R> 10% | The molecule is an efficient antioxidant |
| R<10% | The molecule is a very efficient antioxidant |

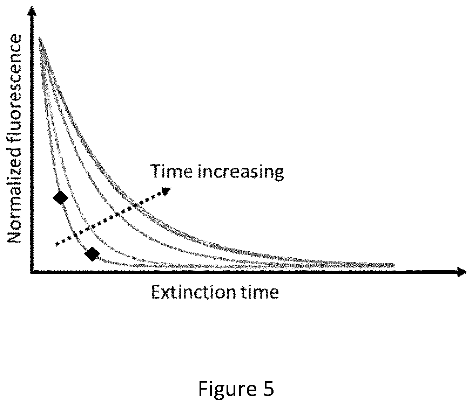

To go further, the measurement described above are performed before, during and after the action of an antioxidant in living skin cells to obtain the results illustrated on Figure 5.

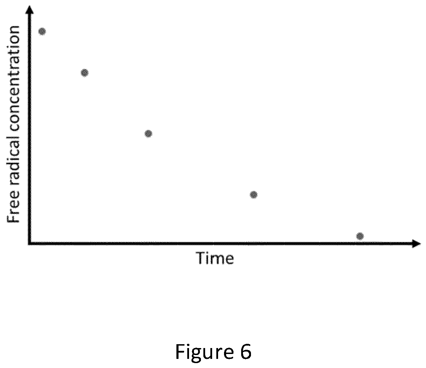

Thus, another example uses the kinetics. Considering the free radical concentration evolution over time as on Figure 6, we measure the antioxidant reaction speed and assess the scavenging efficacy of the antioxidant. A classifying parameter could be the time needed to reach the final concentration.

Thanks to a temporal resolution and a remarkable photostability, kinetics can be performed to evaluate antioxidant activity along time, from seconds to hours and days, when other technology only allow a before/after result.

### Reference List

1. Vangipuram et al.: "Skin punch biopsy explant culture for derivation of primary human fibroblasts". J Vis Exp. 2013 Jul 7;(77):e3779. doi: 10.3791/3779. PMID: 23852182; PMCID: PMC3731437.
2. Tetienne, J. P., Hingant, T., Rondin, L., Cavailles, A., Mayer, L., Dantelle, G., & Jacques, V. (2013). Spin relaxometry of single nitrogen-vacancy defects in diamond nanocrystals for magnetic noise sensing. Physical Review B, 87(23), 235436.
3. Martinez, F. P., Nusantara, A. C., Chipaux, M., Padamati, S. K., & Schirhagl, R. (2020). Nanodiamond Relaxometry-Based Detection of Free-Radical Species When Produced in Chemical Reactions in Biologically Relevant Conditions. ACS sensors, 5(12), 3862-3869.

## Claims

1. A method for assessing antioxidant efficacy of one or more molecule(s) introduced inside or in the vicinity of a cell by measuring, in said cell, free radical concentration, the method comprising the steps of:
- Functionalizing nanodiamonds having Nitrogen-Vacancy (NV) centers with species able to target an organelle within the cell,
- Introducing the functionalized nanodiamonds inside the cell,
- Illuminating NV centers with green light, and
- Measuring red fluorescence of the NV centers in the cell after the action of the molecule(s), and also optionally before and/or during the action of the molecule(s),
wherein red fluorescence of the NV centers depends on the free radical concentration in the cell.

2. A method according to claim 1, wherein the cell is a living cell or a lysed cell.

3. A method according to anyone of the preceding claims, wherein the cell is chosen among skin, liver, kidney, lung, uterus, prostate, testis, tongue, bone marrow, colon, bronchi, muscle, pancreas, brain, eye, blood, urinary bladder and gallbladder cells.

4. A method according to anyone of the preceding claims, wherein said organelle is mitochondria.

5. A method according to anyone of the preceding claims, wherein the step of measuring red fluorescence of the NV centers in the cell is repeated over time to follow a kinetic reaction.

6. A method according to anyone of the preceding claims, wherein the species is chosen as COOH terminating said nanodiamonds linked covalently via amide linkage either with trans-activating transcriptional activator (TAT) peptide or with triphenyl phosphonium (TPP) tags.

7. A device comprising a processor and memory, said memory comprising code that, when executed by said processor, causes the device to perform the method according to any one of the claims 1 to 6.

8. A nanodiamond (ND) having Nitrogen-Vacancy (NV) centers with species able to target an organelle within a cell, said species being chosen as COOH terminating said nanodiamonds linked covalently via amide linkage either with trans-activating transcriptional activator (TAT) peptide or with triphenyl phosphonium (TPP) tags.

9. A process for preparing a nanodiamond (ND) as defined in claim 8, wherein the species is COOH terminating said nanodiamonds linked covalently with trans-activating transcriptional activator (TAT) peptide via amide linkage, said process comprising the steps of:
A1) activating the surface carboxyl groups of the nanodiamond using N-ethyl-N'-(-3-dimethylaminopropyl) carbodiimide hydrochloride (EDC) and N-hydroxysuccinimide (NHS) or Sulfo-NHS, and
A2) reacting the activated carboxyl groups with NH2 terminated TAT peptide to form TAT-ND via amide linkage between the carboxyl and amine groups,
OR
B1) activating the surface carboxyl groups of the nanodiamond using N-ethyl-N'-(-3-dimethylaminopropyl) carbodiimide hydrochloride (EDC) and N-hydroxysuccinimide (NHS) or Sulfo-NHS,
B2) reacting activated carboxyl groups with PEG-diamine to form NH2-PEG-ND,
B3) activating carboxyl groups in TAT peptide using EDC, NHS or sulfo-NHS and reacting them with NH2-PEG-ND to form TAT-PEG-ND.

10. A process for preparing a nanodiamond (ND) as defined in claim 8, wherein the species is COOH terminating said nanodiamonds linked covalently with triphenyl phosphonium (TPP) tags, said process comprising the steps of:
C1) activating the surface carboxyl groups of ND using N-ethyl-N'-(-3-dimethylaminopropyl) carbodiimide hydrochloride (EDC) and N-hydroxysuccinimide (NHS) or Sulfo-NHS,
C2) reacting said activated carboxyl groups with NH2 terminated TPP tag to form ND-TPP via amide linkage.

11. Use of a nanodiamond (ND) as defined in claim 8 for sensing qualitatively or quantitatively free radicals in a cell sample.

## Amended claims

### Amended claims in accordance with Rule 137(2) EPC.

1. A method for assessing antioxidant efficacy of one or more molecule(s) introduced inside or in the vicinity of a cell by measuring, in said cell, free radical concentration, the method comprising the steps of:
- Functionalizing nanodiamonds having Nitrogen-Vacancy (NV) centers with species able to target an organelle within the cell,
- Introducing the functionalized nanodiamonds inside the cell,
- Illuminating NV centers with green light, and
- Measuring red fluorescence of the NV centers in the cell before, during and after the action of the molecule(s),
wherein red fluorescence of the NV centers depends on the free radical concentration in the cell.

2. A method according to claim 1, wherein the cell is a living cell or a lysed cell.

3. A method according to anyone of the preceding claims, wherein the cell is chosen among skin, liver, kidney, lung, uterus, prostate, testis, tongue, bone marrow, colon, bronchi, muscle, pancreas, brain, eye, blood, urinary bladder and gallbladder cells.

4. A method according to anyone of the preceding claims, wherein said organelle is mitochondria.

5. A method according to anyone of the preceding claims, wherein the step of measuring red fluorescence of the NV centers in the cell is repeated over time to follow a kinetic reaction.

6. A method according to anyone of the preceding claims, wherein the species is chosen as COOH terminating said nanodiamonds linked covalently via amide linkage either with trans-activating transcriptional activator (TAT) peptide or with triphenyl phosphonium (TPP) tags.

7. A device comprising a processor and memory, said memory comprising code that, when executed by said processor, causes the device to perform the method according to any one of the claims 1 to 6.

8. A nanodiamond (ND) having Nitrogen-Vacancy (NV) centers with species able to target an organelle within a cell, said species being chosen as COOH terminating said nanodiamonds linked covalently via amide linkage either with trans-activating transcriptional activator (TAT) peptide using polyethylene glycol diamine (NH2-PEG-NH2) or with triphenyl phosphonium (TPP) tags.

9. A process for preparing a nanodiamond (ND) as defined in claim 8, wherein the species is COOH terminating said nanodiamonds linked covalently with trans-activating transcriptional activator (TAT) peptide via amide linkage, said process comprising the steps of:
B1) activating the surface carboxyl groups of the nanodiamond using N-ethyl-N'-(-3-dimethylaminopropyl) carbodiimide hydrochloride (EDC) and N-hydroxysuccinimide (NHS) or Sulfo-NHS,
B2) reacting activated carboxyl groups with PEG-diamine to form NH2-PEG-ND,
B3) activating carboxyl groups in TAT peptide using EDC, NHS or sulfo-NHS and reacting them with NH2-PEG-ND to form TAT-PEG-ND.

10. A process for preparing a nanodiamond (ND) as defined in claim 8, wherein the species is COOH terminating said nanodiamonds linked covalently with triphenyl phosphonium (TPP) tags, said process comprising the steps of:
C1) activating the surface carboxyl groups of ND using N-ethyl-N'-(-3-dimethylaminopropyl) carbodiimide hydrochloride (EDC) and N-hydroxysuccinimide (NHS) or Sulfo-NHS,
C2) reacting said activated carboxyl groups with NH2 terminated TPP tag to form ND-TPP via amide linkage.

11. Use of a nanodiamond (ND) as defined in claim 8 for sensing qualitatively or quantitatively free radicals in a cell sample.


SEQUENCE LISTING

<110>  WAINVAM-E

<120>  METHODS FOR SENSING, MEASURING FREE RADICALS, AND ASSESSING
       ANTIOXIDANT EFFICACY OF A MOLECULE IN A CELL, AND FLUORESCENT
       PROBES USED THEREIN

<130>  BNT230859EP00

<160>  1

<170>  PatentIn version 3.5

<210>  1
<211>  11
<212>  PRT
<213>  Homo sapiens

<400>  1

Tyr Gly Arg Lys Lys Arg Arg Gln Arg Arg Arg
1               5                   10